# FASCICULE DE BREVET EUROPEEN

(11) **EP 2 271 357 B1**
(45) Date de publication et mention de la délivrance du brevet: **18.03.2015**
(21) Numéro de dépôt: 09742231.5
(22) Date de dépôt: 02.04.2009
(51) Int. Cl.: A61Q 19/00, A61K 8/64

(54) **UTILISATION COSMETIQUE DE PROTEINES DE TYPE ANNEXINE II POUR LE TRAITEMENT DE LA SECHERESSE CUTANEE**
KOSMETISCHE ANWENDUNG DES PROTEINS ANNEXIN II ZUR BEHANDLUNG VON HAUTTROCKENHEIT
COSMETIC USE OF ANNEXIN II-TYPE PROTEINS FOR TREATING DRYNESS OF THE SKIN

(30) Priorité: 04.04.2008 FR 0852281; 30.04.2008 US 49004 P
(43) Date de publication de la demande: 12.01.2011
(73) Titulaire: L'Oréal, 75008 Paris (FR)
(72) Inventeur: BERNARD, Dominique, 92170 VANVES (FR); SIMONETTI, Lucie, F-94300 Vincennes (FR); CASTIEL, Isabelle, F-06200 Nice (FR)
(74) Mandataire: Le Coupanec, Pascale A.M.P.
(86) Numéro de dépôt international: PCT/FR2009/050563
(87) Numéro de publication internationale: WO 2009/136033

(56) Documents cités:
- WO-A-2005/027965
- DATABASE WPI Week 200805 Thomson Scientific, London, GB; AN 2008-A66152 XP002571771 & JP 2007 246438 A (NIPPON MENARD KESHOHIN KK) 27 septembre 2007 (2007-09-27)
- DATABASE WPI Week 200736 Thomson Scientific, London, GB; AN 2007-389966 XP002516487 & WO 2007/023808 A (KIRIN BEER KK) 1 mars 2007 (2007-03-01)

## Description

La présente invention a pour objet un procédé, notamment cosmétique, non invasif, pour caractériser l'état de sécheresse de la surface d'un épithélium, notamment dun épiderme, comprenant au moins la caractérisation qualitative ou quantitative de l'expression et/ou de l'activité biologique d'un polypeptide conforme à l'invention c'est-à-dire l'annexine II, de l'un de ses dérivés ou fragments.

L'invention concerne également un procédé, notamment cosmétique, non invasif, pour caractériser efficacité d'un traitement cosmétique ou thérapeutique visant à suppléer aux signes de sécheresse cutanée comprenant au moins la caractérisation qualitative ou quantitative de l'expression et/ou de l'activité biologique d'un polypeptide conforme à l'invention, c'est-à-dire de l'annexine II. de l'un de ses dérivés ou fragments.

L'épiderme est un épithélium, conventionnellement divisé en une couche basale de kératinocytes contenant, notamment, des cellules souches cutanées et constituant la couche germinative de l'épiderme, une couche dite épineuse constituée de plusieurs couches de cellules polyhédriques disposées sur la couche basale, une couche dite granuleuse comprenant une à trois couches dites de cellules aplaties contenant des inclusions cytoplasmiques distinctes, les grains de kératohyaline, et enfin, un ensemble de couches supérieures, appelé couche cornée (ou *stratum corneum*) constituée de kératinocytes au stade terminal de leur différenciation appelés cornéocytes.

Le *stratum corneum*, de par sa solidité et sa structure stratifiée compacte, assure une fonction barrière : il s'oppose notamment à la perte en eau transcutanée, aussi appelée « perte insensible en eau ».

Ainsi, l'une des fonctions du *stratum corneum* est de capter et retenir l'eau contenue dans l'épiderme, et toute altération de sa structure et/ou de sa fonction pourra se traduire par des modifications de l'hydratation cutanée.

L'hydratation est apportée à la peau par l'eau des couches profondes et par la sueur.

Un déséquilibre de l'hydratation de la peau peut se traduire par de profondes conséquences tout autant physiologiques que cosmétiques.

Des troubles de l'hydratation cutanée, et notamment une sécheresse cutanée, sont souvent observés avec l'âge. Toutefois, de tels états peuvent également se manifester chez des sujets jeunes.

L'état de sécheresse cutanée peut être d'origine acquise ou constitutionnelle non pathologique ou elle peut avoir une origine constitutionnelle pathologique.

De nombreux facteurs extérieurs peuvent entraîner l'assèchement de la peau ou aggraver cet état. Parmi ces facteurs, on peut citer les conditions climatiques telles que le froid ou le vent, les rayons solaires, l'exposition à certains agents chimiques ou thérapeutiques.

Sur le plan physiologique, une peau sèche est souvent associée à une baisse du taux d'hydratation cutanée ainsi qu'à une modification du processus de maturation du *stratum corneum* ayant pour signe le plus visible l'apparition de squames à la surface cutanée.

Sur le plan sensoriel, une peau sèche peut être caractérisée par une sensation de tiraillement et/ou de tension cutanée.

Différentes méthodes existent pour évaluer une peau sèche.

Un premier type d'évaluation, effectuée d'un point de vue strictement visuel, s'appuie sur un atlas photographique et utilise une échelle de 0 à 4 pour noter le score. La valeur 0 correspond à une peau parfaitement normale tandis que la valeur 4 correspond à une peau très sèche. Cette méthode de nature subjective, présente l'inconvénient de nécessiter la présence de symptômes cutanés visibles.

Un second type d'évaluation repose sur des mesures biophysiques.

Ces méthodes sont basées pour la plupart sur les propriétés électriques de la peau, c'est-à-dire la capacitance, la conductance et l'impédance cutanée. C'est le cas par exemple de la mesure de l'indice d'hydratation de la couche cornée par coméométrie, fondée sur l'aptitude de la peau à conduire un courant électrique. Celle-ci peut s'effectuer par différents appareils commercialisés, tels que le coméomètre de la société COURAGE & KHAZAKA.

Enfin, d'autres types d'évaluation, parfois qualifiés de techniques « morphométriques », se rapportent à l'analyse du microrelief cutané ou de l'état des cellules à la surface cutanée, telle que l'évaluation de la « desquamation » en effectuant des prélèvements par « stripping » à l'aide d'adhésif de type « D'squam » de la société CU-DERM.

Ces méthodes présentent l'inconvénient de devoir être combinées entre elles pour pouvoir donner une mesure fiable de l'état de sécheresse de la peau.

Par ailleurs, elles présentent également l'inconvénient de ne pas donner d'indice sur l'origine de la peau sèche et donc sur les moyens de la corriger.

Par conséquent, il existe un besoin de pouvoir disposer d'un nouveau marqueur, notamment d'un marqueur biologique, propice à donner une mesure fiable d'un état de l'épithélium, et notamment d'un état de sécheresse.

Il existe donc un besoin de pouvoir disposer de nouveaux outils permettant de promouvoir l'intégrité et la maturation du *stratum corneum* ainsi que d'évaluer son état.

Il existe également un besoin de pouvoir disposer de nouveaux outils et/ou de nouvelles cibles cosmétiques et/ou dermatologiques pouvant être mis en oeuvre aux fins de prévenir et/ou soulager les signes physiologiques et/ou sensoriels liés à une sécheresse d'un épithélium, et notamment d'un épiderme.

Il existe également un besoin de disposer de nouvelles cibles cosmétiques ou dermatologiques pour le traitement de l'état de sécheresse d'un épithélium et notamment d'un épiderme.

Il existe également un besoin de disposer de nouveaux outils, notamment de nouvelles molécules, pour le traitement et/ou la prévention d'un état de sécheresse d'un épithélium, et notamment de l'épiderme.

La présente invention a pour objet de satisfaire à ces besoins.

La présente invention résulte plus particulièrement de la caractérisation par les inventeurs d'une augmentation du niveau d'expression de la protéine annexine II dans le *stratum corneum* d'un épiderme humain sec, en comparaison avec le niveau dans un *stratum corneum* d'épiderme normal. Et plus particulièrement, l'invention découle de l'observation d'une expression augmentée de l'annexine II dans un épiderme humain âgé et sec par rapport à un épiderme jeune ou âgé et à l'hydratation normale.

L'annexine II est une protéine de 339 acides aminés (SEQ ID NO 2) qui, pour assurer ses fonctions, peut s'associer à la protéine S100A10 pour former un hétérotétramère composé de deux sous-unités de chacune des protéines.

Elle appartient à une famille de protéines de liaison aux phospholipides calcium-dépendantes, indiquant une localisation des annexines au niveau de la membrane plasmique. De par cette localisation particulière, on prête aux annexines un rôle dans l'organisation des événements de fusion membranaire, de type exocytose et endocytose. Ces protéines interviennent dans diverses voies de signalisation, notamment dans celle du calcium.

L'annexine II est une protéine ubiquitaire dont l'expression est particulièrement abondante dans la peau. En raison d'une activité de canal calcique montrée *in vitro*, il a été suggéré que lors de la différenciation terminale des kératinocytes, l'annexine II puisse intervenir dans la régulation du flux calcique.

Dans une étude portant sur le vieillissement cutané, Gromov *et al*. n'ont pas observé de variation de l'expression de la protéine annexine II avec l'âge (Mol.Coll Proteomics, 2003 Feb. ; 2(2) : 70-84).

A la connaissance des inventeurs, l'annexine II n'avait pas jusqu'ici été identifiée comme étant une protéine dont l'expression varie en fonction de la typologie de la peau, à savoir un niveau d'expression accru dans un *stratum corneum* humain sec par rapport à celui présent dans un *stratum corneum* humain normal.

Ainsi, contre toute attente, l'annexine II s'avère être également un marqueur potentiel de l'état physiologique de la peau, notamment en terme de sécheresse. En effet, comme il ressort des essais figurant ci-après, les inventeurs ont constaté de manière inattendue, d'une part une expression de cette protéine au niveau du *stratum corneum*, et d'autre part une augmentation significative de son expression dans un *stratum corneum* prélevé sur une peau sèche, en comparaison avec un *stratum corneum* prélevé sur une peau normale.

Ainsi, le présent texte décrit une utilisation cosmétique ou encore non thérapeutique, d'une quantité efficace d'au moins un polypeptide dérivé de l'annexine II et, notamment, de séquence d'acides aminés codée par une séquence d'acides nucléiques représentée en tout ou partie par une séquence représentée par SEQ ID NO 1, un analogue de celle-ci ou un fragment de celle-ci, d'au moins une séquence nucléique codant pour un tel polypeptide ou d'au moins un agent modulateur de l'activité, de la stabilité ou de l'expression d'un tel polypeptide, à titre d'agent utile , pour prévenir et/ou traiter les signes de sécheresse cutanée, en particulier à prévenir et/ou traiter une déshydratation d'un épiderme.

Le présent texte décrit également une utilisation d'une quantité efficace d'au moins un polypeptide dérivé de l'annexine II et, notamment, de séquence d'acides aminés codée par une séquence d'acides nucléiques représentée en tout ou partie par une séquence représentée par SEQ ID NO 1, un analogue de celle-ci ou un fragment de celle-ci, d'au moins une séquence nucléique codant pour un tel polypeptide ou d'au moins un agent modulateur de l'activité, de la stabilité ou de l'expression d'un tel polypeptide pour la préparation d'une composition, notamment thérapeutique, destinée à prévenir et/ou traiter les signes de sécheresse cutanée, en particulier à prévenir et/ou traiter une déshydratation d'un épiderme.

Par signes de sécheresse cutanée, on entend non seulement toutes les modifications de l'aspect extérieur de la peau dues notamment à la déshydratation de l'épiderme, telles que l'aspect rugueux et écailleux, ainsi que la souplesse diminuée, mais aussi les sensations liées au phénomène de sécheresse, telles que les démangeaisons et/ou les tiraillements. Il peut en effet arriver que ces sensations soient éprouvées par une personne sans qu'aucun symptôme visuel ne soit nécessairement perceptible.

Une telle utilisation peut être plus particulièrement destinée à prévenir et/ou traiter les signes de sécheresse cutanée acquise ou constitutionnelle non-pathologique.

Au sens de la présente invention, on entend désigner par l'expression « quantité efficace » la quantité minimale nécessaire à l'observation de l'effet attendu, à savoir un effet cosmétique ou un effet thérapeutique, étant entendu que les quantités efficaces nécessaires à l'obtention d'un effet cosmétique ou d'un effet thérapeutique peuvent être, le cas échéant, identiques ou différentes.

Au sens de l'invention, on entend désigner par « utilisation cosmétique » une utilisation destinée, principalement, à procurer un effet esthétique et/ou du confort.

Au sens de l'invention, on entend désigner par « composition thérapeutique » une composition destinée à procurer un effet prophylactique ou curatif au regard de troubles épithéliaux, et notamment épidermiques, reconnus comme traduisant un état pathologique.

Par « prophylactique » ou « préventif » au sens de l'invention, on entend la diminution du risque de survenue d'un phénomène, par exemple une pathologie.

Une composition décrite dans le présent texte peut, en particulier, être destinée à prévenir et/ou traiter une sécheresse d'un épiderme, et notamment un défaut d'hydratation du *stratus corneum*.

Une composition décrite dans le présent texte peut, en particulier, être destinée à prévenir et/ou traiter des sensations de démangeaisons et/ou de tiraillements d'un épithélium sec.

Le présent texte décrit également l'utilisation d'au moins un polypeptide conforme à l'invention, ou d'au moins une séquence d'acides nucléiques codant pour ledit polypeptide, à titre d'outil de caractérisation *in vitro* ou *ex vivo* dun état de sécheresse d'un épithélium, et notamment dun épiderme.

Selon un de ses premiers aspects, la présente invention concerne un procédé, notamment cosmétique, non invasif, pour caractériser l'état de sécheresse de la surface d'un épithélium, notamment d'un épiderme, comprenant au moins la caractérisation qualitative ou quantitative de l'expression et/ou de l'activité biologique dun polypeptide conforme à l'invention c'est-à-dire l'annexine II, de l'un de ses dérivés ou fragments.

Plus précisément, la présente invention concerne un procédé de caractérisation d'un état de sécheresse d'un épithélium comprenant au moins les étapes consistant à :
a) déterminer dans un échantillon dudit épithélium, une teneur d'un polypeptide de séquence d'acides aminés codée par une séquence d'acides nucléiques représentée par une séquence représentée par SEQ ID NO 1, un analogue de celle-ci ayant au moins 85 % d'homologie de séquence ainsi qu'une activité biologique de même nature, ou un fragment de celle-ci comprenant au moins 6 acides aminés consécutifs dudit polypeptide ainsi qu'une activité biologique de même nature ou d'une séquence dacides nucléiques codant pour ledit polypeptide, et
b) comparer ladite teneur déterminée à l'étape a) à une valeur de référence.

La donnée ou valeur de référence peut être obtenue par exemple à partir d'au moins un épithélium, notamment un épiderme, distinct de celui faisant l'objet de la caractérisation, et dont l'état est connu. A titre d'épiderme de référence, il peut s'agir soit d'un épiderme dun second sujet ayant une peau normale et distincte d'un premier sujet chez qui la caractérisation est effectuée, soit d'une région de l'épiderme du même sujet chez qui la caractérisation est effectuée, mais choisie dans une zone cutanée présentant une hydratation physiologique.

La présente invention vise selon un autre de ses aspects un procédé, notamment cosmétique, non invasif, pour caractériser l'efficacité d'un traitement cosmétique ou thérapeutique visant à suppléer aux signes de sécheresse cutanée comprenant au moins la caractérisation qualitative ou quantitative de l'expression et/ou de l'activité biologique d'un polypeptide conforme à l'invention, cest-à-dire de l'annexine II, de l'un de ses dérivés ou fragments.

Plus précisément, la présente invention concerne un procédé non thérapeutique, pour mettre en évidence un effet d'un traitement susceptible de faire régresser des signes de sécheresse d'un épithélium, notamment des sensations de démangeaisons et/ou de tiraillements, chez un individu, comprenant au moins les étapes consistant à :
a) effectuer, avant le traitement, au moins une première détermination, dans un premier échantillon d'un épithélium prélevé chez ledit individu, d'une activité biologique et/ou de l'expression d'un polypeptide de séquence d'acides aminés codée par une séquence d'acides nucléiques représentée par une séquence représentée par SEO ID NO 1, un analogue de celle-ci ayant au moins 85 % d'homologie de séquence ainsi qu'une activité biologique de même nature, ou un fragment de celle-ci comprenant au moins 6 acides aminés consécutifs dudit polypeptide ainsi qu'une activité biologique de même nature ou de l'expression d'une séquence d'acides nucléiques codant pour ledit polypeptide.
b) effectuer, après le traitement, au moins une seconde détermination, dans un second échantillon d'un épithélium prélevé chez ledit individu, de ladite activité biologique et/ou de ladite expression dudit polypeptide ou de ladite expression de ladite séquence d'acides nucléiques déterminées à l'étape a), et
c) comparer les première et deuxième déterminations, notamment afin d'en déduire une information relative à un effet au moins du traitement.

La valeur ou donnée de référence peut être une donnée obtenue à partir de épithélium, notamment de l'épiderme, à soumettre au traitement, antérieurement à l'administration dudit traitement ou dans un délai chronologique plus court au regard de la date de début de traitement.

Comme il ressort de la description qui suit, les procédés selon l'invention sont particulièrement avantageux dans la mesure où leur mise en oeuvre ne requiert pas d'opération invasive.

Les procédés de invention peuvent être effectués *in vitro, ex vivo* ou *in vivo.*

En effet, la localisation par les inventeurs du nouveau biomarqueur de sécheresse qu'est l'annexine II, dans le *stratum corneum* rend possible une caractérisation quantitative ou qualitative de l'expression de cette protéine par simple prélèvement topique. La méthode de prélèvement peut par exemple être une technique de type « stripping » consistant à appliquer sur l'épithélium considéré, tel qu'un épiderme, une portion de ruban adhésif. En décollant ce ruban adhésif, une fraction de l'épithélium, par exemple une fraction épidermique, est prélevée. Après extraction protéique, celle-ci est analysée par des méthodes conventionnelles, telles que le dosage immuno-enzymatique ou plus particulièrement une analyse Western-Blot.

### PEAU SECHE

Une peau sèche apparaît rugueuse au toucher et apparaît couverte de squames et se manifeste pour l'essentiel par une sensation de tiraillements et/ou de tension.

La peau sèche est en effet accompagnée d'un trouble de la desquamation et présente différents stades en fonction de la sévérité de cette desquamation. Lorsque la peau est légèrement sèche, ces squames sont abondantes mais peu visibles à l'oeil nu, l'élimination s'effectue cornéocyte par cornéocyte. Elles sont de moins en moins nombreuses mais de plus en plus visibles à l'oeil nu lorsque ce désordre s'aggrave, les amas peuvent comprendre plusieurs centaines de cornéocytes, représentant ainsi des amas plus ou moins grands, appelées squames.

L'origine de cette sécheresse cutanée peut être de type constitutionnel ou acquis.

Dans le cas de la peau sèche constitutionnelle, on peut distinguer deux catégories : les peaux pathologiques et les peaux non pathologiques.

Les peaux sèches constitutionnelles pathologiques sont essentiellement représentées par la dermatite atopique et les ichthyoses. Elles sont quasiment indépendantes des conditions extérieures et ont pour origine des modifications génétiques connues ou inconnues. Parmi les modifications génétiques connues affectant l'hydratation cutanée on peut citer par exemple des modifications du gène de la Transglutaminase 1 ou celles du gène de la Filaggrine.

Dans le cas des peaux sèches constitutionnelles non pathologiques, la sévérité de l'état de sécheresse peut, pour sa part, dépendre de facteurs extérieurs. Rentrent dans cette catégorie de peau, la peau sénile (caractérisée par une diminution générale du métabolisme cutané avec l'âge), la peau fragile (très sensible aux facteurs extérieurs et souvent accompagnée d'érythème et de rosacée) et la xérose vulgaire (d'origine génétique probable et se manifestant en priorité sur le visage, les membres et le dos des mains).

Dans le cas de peau sèche acquise, l'intervention de paramètres extérieurs tels que l'exposition aux agents chimiques, à des conditions climatiques difficiles, aux rayons solaires ou bien encore certains traitements thérapeutiques (rétinoïdes, par exemple) est déterminante. Sous ces influences extérieures, l'épiderme peut devenir alors momentanément et localement sec. Cela peut concerner tout type d'épiderme.

Quelle qu'en soit l'origine, une peau atteinte de sécheresse cutanée présente, généralement, les signes suivants, à savoir un aspect rugueux au toucher et écailleux, ainsi qu'une souplesse et une élasticité diminuées.

La peau sèche, également dénommée « xérose », peut apparaître à n'importe quel âge, et n'être pas liée à une condition pathologique. On parlera dans ce cas de sécheresse « acquise ».

Cependant, la xérose devient plus fréquente et gênante avec l'âge, notamment chez les femmes. On parle alors de xérose sénile. Par ailleurs, les femmes subissent généralement une aggravation de l'assèchement cutané lors de la ménopause, probablement du fait du dérèglement hormonal caractéristique de ce phénomène. Les zones les plus affectées sont la partie inférieure des jambes, la partie dorsale des avant-bras et les mains.

Comme mentionné précédemment, la sécheresse acquise peut subir l'influence de facteurs extérieurs. Par exemple, l'apparition de la peau sèche peut être favorisée par un temps froid, sec et hivernal. On parle alors de xérose hivernale. La sécheresse cutanée peut également être induite par un stress exogène, d'origine chimique, par exemple de type détergent anionique, ou encore d'origine mécanique (frottement, rasage).

Bien qu'aucune étude n'ait démontré une incidence de la sécheresse sur l'origine et la formation des rides et ridules qui sont essentiellement attribuables au vieillissement, sur le plan visuel, une peau sèche rend celles-ci plus apparentes.

Par ailleurs, sur le plan sensoriel, la sécheresse cutanée est caractérisée par une sensation de tiraillements et/ou de démangeaisons. Pour des raisons évidentes, ces manifestations sont non seulement source d'inconforts, voire de douleurs, mais également d'une apparence inesthétique.

Ainsi, il demeure un besoin de disposer de nouveaux actifs susceptibles d'exercer une action bénéfique dans le traitement de la sécheresse cutanée non seulement sur le plan thérapeutique, mais également sur le plan esthétique.

### DEFINITION POLYPEPTIDE

Selon un mode de réalisation, un polypeptide convenant à l'invention peut avoir une séquence d'acides aminés représentée en tout ou partie par une séquence représentée par SEQ ID NO 2, ou un analogue de celle-ci, ou un fragment de celle-ci.

Au sens de la présente invention, on entend désigner de manière générale, sauf indication contraire, par l'annexine II la séquence (SEQ ID NO 2) de la protéine ayant ou non subi des modifications post-traductionnelles.

Parmi ces modifications post-traductionnelles, on peut citer, en particulier, des phosphorylations sur les acides aminés en position 18, 19, 24, 26 et 30 de la protéine.

Il est par ailleurs connu que la séquence primaire d'un polypeptide, c'est-à-dire l'enchaînement des acides aminés, détermine des sites spécifiquement reconnus par des enzymes de type protéase, telles que la trypsine qui, une fois la reconnaissance de ces sites effective, vont induire le clivage du polypeptide par protéolyse. Cette protéolyse résulte en la génération de divers peptides, ou fragments de protéolyse, de l'annexine II.

Les inventeurs ont détecté la présence de tels peptides dans le *stratum corneum*.

En conséquence, l'invention s'étend également aux fragments de protéolyse de l'Annexine II.

Ainsi, selon un mode de réalisation particulier, un polypeptide convenant à l'invention peut avoir une séquence d'acides aminés choisie parmi SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24, SEQ ID NO 25, SEQ ID NO 26, SEQ ID NO 27, SEQ ID NO 28, SEQ ID NO 29, SEQ ID NO 30, SEQ ID NO 31, SEQ ID NO 32, SEQ ID NO 33, SEQ ID NO 34, SEQ ID NO 35, SEQ ID NO 36, SEQ ID NO 37, SEQ ID NO 38, SEQ ID NO 39, SEQ ID NO 40, SEQ ID NO 41, SEQ ID NO 42 et SEQ ID NO 43, et leurs mélanges.

Par « analogue d'un polypeptide », on entend désigner tout polypeptide présentant une homologie de séquence, en particulier vis-à-vis d'une des séquences caractéristiques dudit polypeptide, ainsi qu'une activité biologique de même nature.

Ce composé peut être un agent peptidomimétique.

L'homologie est d'au moins 85 %, et peut être par exemple d'au moins 90 %, et par exemple d'au moins 95 %. L'homologie peut être déterminée par comparaison visuelle ou au moyen de tout outil informatique généralement utilisé dans le domaine, tel que les programmes BLAST disponibles sur www.ncbi.nlm.nih.gov et utilisés avec les paramètres configurés par défaut.

L'homologie de séquence peut résulter de modifications issues de mutation ou de variation dans les séquences des peptides selon l'invention provenant soit de la délétion ou de l'insertion d'un ou plusieurs acides aminés, soit de la substitution d'un ou plusieurs acides aminés dans les séquences caractéristiques d'un polypeptide selon l'invention.

Au sens de l'invention, on entend désigner par « fragment de polypeptide » toute portion d'un polypeptide conforme à l'invention comprenant au moins 6, en particulier au moins 8, et plus particulièrement au moins 12 acides aminés consécutifs dudit polypeptide, et une activité biologique sensiblement similaire.

Par « séquence caractéristique du polypeptide », on entend viser notamment au regard de l'annexine II, la séquence représentée par SEQ ID NO 2.

De manière générale, les analogues polypeptidiques peuvent comprendre des substitutions conservatrices par rapport à la séquence d'acides aminés du polypeptide naturel.

Plusieurs de ces modifications peuvent être combinées.

A titre d'exemple de mutations que l'on peut considérer dans la présente invention, il peut être mentionné, de manière non exhaustive, le remplacement d'un ou plusieurs résidus d'acides aminés par des résidus d'acides aminés ayant un indice hydropathique similaire sans pour autant affecter de manière sensible les propriétés biologiques du polypeptide, et notamment son activité biologique, telle que son activité de stimulation de la prolifération et/ou de la migration et/ou de la différenciation terminale des kératinocytes ou de la stimulation de la synthèse des protéoglycannes au niveau d'un épithélium, et en particulier de l'épiderme.

L'indice hydropathique est un indice attribué aux acides aminés en fonction de leur hydrophobicité et de leur charge (Kyte et al. (1982), J. Mol. Biol., 157 : 105).

Un polypeptide ou analogue également visé par la présente invention peut être un polypeptide ayant subi une ou plusieurs modification(s) post-traductionnelle(s).

Par « modification(s) post-traductionnelle(s) », on entend englober l'ensemble des modifications qu'un peptide ou une protéine est susceptible de subir à l'issue de sa synthèse dans une cellule, telles que, par exemple, une ou des phosphorylation(s), une ou des thiolation(s), une ou des acétylation(s), une ou des glycosylation(s), une ou des lipidation(s), telles qu'une farnésylation ou une palmitoylation, un réarrangement structural de type formation de ponts disulfures et/ou de type clivage à l'intérieur de la séquence peptidique.

L'analogue présente, par ailleurs, sensiblement la même activité biologique que le polypeptide naturel.

Selon un mode de réalisation un polypeptide convenant à la mise en oeuvre de l'invention peut également être un polypeptide naturel ou synthétique, le cas échéant susceptible d'être obtenu après lyse enzymatique ou chimique de l'annexine II ou par synthèse chimique ou biologique ou par extraction à partir d'un tissu biologique, comme par exemple la peau, exprimant naturellement ce polypeptide ou après transfection de celui-ci, ainsi que les différentes formes post-traductionnelles de celui-ci, ou encore tout polypeptide naturel ou synthétique dont la séquence comprend totalement ou partiellement (en tout ou partie) une séquence d'acides aminés précitée, par exemple les variants et les analogues.

L'homme de l'art peut obtenir un polypeptide conforme à l'invention au moyen de procédés à base d'ADN recombinant, comme par exemple, ceux décrits dans le manuel «Molecular Cloning - A Laboratory Manual » (2ème édition), Sambrook et al., 1989, Vol. I-III, Coldspring Harbor Laboratory, Coldspring Harbor Press, NY, (Sambrook).

Selon un autre mode de réalisation, un polypeptide convenant à la mise en oeuvre de l'invention peut également être un polypeptide tel que défini précédemment dans lequel un résidu au moins a été remplacé par un résidu d'acide aminé d'indice hydropathique similaire, comme défini précédemment.

Selon un autre mode de réalisation, un polypeptide convenant à la mise de l'invention peut également être un polypeptide tel que défini précédemment, fusionné avec un autre polypeptide, un agent de ciblage hydrophile ou hydrophobe, un précurseur de bioconversion, un agent de marquage luminescent, radioactif ou colorimétrique.

De manière non limitative, on peut citer comme exemple de composés susceptibles d'être couplés avec un polypeptide conforme à l'invention des protéines fluorescentes comme la « Green Fluorescent Protein », des composés chimiques fluorescents tels que la rhodamine, la fluorescéine, ou le Texas Red^{®}, des composés phosphorescents, des éléments radioactifs, tels que ³H, ¹⁴C, ^{3s}S, ¹²¹I ou ¹²⁵I, ou des agents de marquage colorimétrique comme les substrats chromogènes sensibles à l'action de la galactosidase, de la peroxydase, de la chloramphénicol acétyltransférase, de la luciférase ou de la phosphatase alcaline.

Selon la nature des composés susceptibles d'être couplés avec un polypeptide conforme à l'invention, le couplage peut être opéré par des procédés chimiques, notamment au moyen de fonctions chimiques réactives ou par des procédés de biologie moléculaire connus de l'homme de l'art.

### DEFINITION SEQUENCES ACIDES NUCLEIOUES

Selon un mode de réalisation, la présente invention concerne également des séquences d'acides nucléiques codant pour un polypeptide de l'invention et leur mise en oeuvre dans les différents procédés conformes à l'invention.

Ainsi, le présent texte décrit également à l'utilisation de séquences d'acides nucléiques, notamment d'acides désoxyribonucléiques, ou d'acides ribonucléiques codant pour un polypeptide conforme à l'invention, notamment les séquences correspondant au moins à une séquence d'acides nucléiques représentée par SEQ ID NO 1, des analogues de celle-ci ou un fragment de celle-ci pour la préparation d'une composition décrite dans le présent texte.

Au sens de la présente invention, on entend désigner par « fragment de séquence d'acides nucléiques », une séquence d'acides nucléiques codant pour tout ou partie d'un polypeptide conforme à l'invention, ou un analogue de celui-ci, et en particulier une séquence d'acides nucléiques représentée par SEQ ID NO 1 ou un analogue de celle-ci.

Par « analogue d'une séquence d'acides nucléiques », on entend désigner toute séquence d'acides nucléiques, éventuellement résultant de la dégénérescence du code des acides nucléiques, et codant pour un polypeptide de séquence identique ou analogue à celle du polypeptide codée par ladite séquence d'acides nucléiques.

Les séquences d'acides nucléiques peuvent être issues de toutes origines possibles, à savoir soit animale, en particulier de mammifères et encore plus particulièrement humaine, soit végétale, soit de micro-organismes (virus, phages, bactéries entre autres) ou encore de champignons, sans préjuger du fait qu'elles soient présentes de manière naturelle ou non dans ledit organisme d'origine.

En l'occurrence, le présent texte décrit également l'utilisation de fragments d'acides nucléiques isolés et purifiés codant pour les polypeptides considérés selon l'invention.

Une séquence d'acides nucléiques conforme à l'invention peut comprendre une séquence sens, anti-sens ou interférentielle correspondant à une séquence codant pour un polypeptide conforme à l'invention.

Ainsi, le présent texte décrit également à l'utilisation de séquences d'acides nucléiques, notamment d'acides désoxyribonucléiques, ou d'acides ribonucléiques, codant pour un polypeptide conforme à l'invention.

Les séquences d'acides nucléiques selon l'invention peuvent notamment être utilisées pour préparer les séquences d'acides ribonucléiques correspondantes, sens ou anti-sens.

Le présent texte décrit également l'utilisation de tout polynucléotide, de séquence d'acides ribonucléiques ou désoxyribonucléiques, comprenant une séquence sens ou anti-sens, notamment « Small interferent RNA » (SiRNA) correspondant au moins à la séquence d'acides nucléiques SEQ ID NO 1 ou un analogue de celle-ci.

### AGENT MODULATEUR

Le présent texte décrit l'utilisation d'un agent modulateur de l'expression et/ou de la stabilité et/ou de l'activité d'un polypeptide conforme à l'invention.

Au sens de l'invention, on entend par « moduler », au regard d'un effet donné, l'action de stimuler ou d'inhiber cet effet.

Au sens de la présente invention, on entend par l'expression « agent modulateur ou composé chimique ou biologique apte à moduler l'activité biologique et/ou l'expression » tout composé apte à agir, directement ou indirectement, sur au moins un polypeptide conforme à l'invention, ou une séquence d'acides nucléiques codant pour celui-ci, ou sur un élément d'une voie de signalisation intra ou extra-cellulaire, ou d'une voie métabolique, impliquant ledit polypeptide, ou sur un élément impliqué dans la régulation de la transcription et/ou de la traduction d'une séquence d'acides nucléiques codant pour ledit polypeptide, ainsi que dans la régulation de la stabilité de celui-ci.

Par « activité biologique », on entend désigner notamment au regard de l'annexine II, l'activité biologique de la protéine représentée par la séquence SEQ ID NO 2.

Cet agent modulateur peut être un agent activateur ou inhibiteur de l'expression d'un polypeptide de l'invention, ou encore un agent régulant la stabilité dudit polypeptide.

A titre illustratif et non limitatif, parmi les agents régulant la stabilité, peuvent être notamment cités des composés stimulant la dégradation protéolytique, tels que des protéases, des agents chélateurs d'ions, des dérivés sulfoniques, des dérivés d'urée, des agents réducteurs, des alpha ou beta-hydroxyacides, l'acide ascorbique ou la nicotinamide.

Plus particulièrement, l'agent modulateur peut être un inhibiteur de l'expression des polypeptides selon l'invention.

L'argent modulateur peut être un agent diminuant la stabilité des polypeptides conformes à l'invention, en stimulant leur dégradation protéolytique.

Il est entendu que l'ensemble des compositions cosmétiques ou thérapeutiques considérées décrites dans le présent texte mettent en oeuvre un milieu physiologiquement acceptable.

Au sens de la présente invention, on entend désigner par « milieu physiologiquement acceptable », un milieu convenant à l'application d'une composition sur un épithélium ou une matière kératinique, telle que la peau, le cuir chevelu, les lèvres, les muqueuses et les fibres kératiniques comme les cheveux, les ongles et les poils, ou le cas échéant par voie orale ou parentérale.

Au sens de la présente invention, on entend désigner par « thérapeutique » une composition pouvant être mise en oeuvre dans le cadre d'un traitement prophylactique et/ou curatif, ou d'une méthode d'évaluation d'un état de sécheresse d'un épithélium, et notamment de l'épiderme.

Une composition cosmétique ou thérapeutique selon le présent texte peut comprendre, en outre, au moins un agent actif cosmétique et/ou thérapeutique.

Comme exemples d'agents actifs utilisables, il peut être fait mention d'huiles cosmétiques, telles que les huiles de silicone, les huiles végétales de type triglycérides, les huiles hydrocarbonées telles que l'huile de Parleam et les esters d'acides gras et d'alcool gras.

Il peut également être possible d'utiliser d'autres agents actifs permettant d'améliorer l'état de la peau, tels que des actifs hydratants ou humidifiants ou des agents actifs permettant d'améliorer la barrière lipidique naturelle, tels que des céramides, des sulfates de cholestérol et/ou des acides gras, et leurs mélanges.

Il peut également être possible d'utiliser des enzymes ayant une activité sur la peau, telles que des protéases, des lipases, des glucosidases, des amidases, des cérébrosidases et/ou des mélanases, et leurs mélanges.

Comme autres exemples d'agents actifs convenant figurent : des actifs analgésiques, des actifs anti-levures, des actifs anti-bactériens, des actifs antiparasitaires, des actifs antifongiques, des actifs antiviraux, des actifs anti-inflammatoires stéroïdiens, des actifs anesthésiques, des actifs antiprurigineux, des actifs kératolytiques, des actifs anti-radicaux libres, des actifs anti-séborrhéiques, des actifs anti-pelliculaires, des actifs anti-acnéiques, des actifs visant à prévenir le vieillissement de la peau et/ou à améliorer son état, des actifs anti-dermatites, des actifs anti-irritants, des actifs immuno-modulateurs, des actifs pour le traitement de la peau sèche, des actifs anti-transpirants, des actifs anti-psoriasiques, des actifs protecteurs contre les UV, des actifs anti-histaminiques, des actifs cicatrisants, des actifs auto-bronzants, des antioxydants tels que le thé vert ou des fractions actives de celui-ci, la glycérine, la laponite, la caféine, des huiles essentielles aromatiques, des colorants, des actifs dépigmentants, des liporégulateurs, des actifs adoucissants, rafraîchissants, déodorants, insensibilisants, blanchissants, nourrissants, des actifs diminuant la différenciation et/ou la prolifération et/ou la pigmentation cutanée, et leurs mélanges.

D'une manière générale, toute composition décrite dans le présent texte peut être appliquée sur la peau (sur toute zone cutanée du corps) ou sur les muqueuses (buccale, jugale, gingivale, génitale, conjonctivale,..).

De façon connue, une composition cosmétique peut contenir également des adjuvants habituels dans le domaine cosmétique, tels que des gélifiants hydrophiles ou lipophiles, des additifs hydrophiles ou lipophiles, des conservateurs, des antioxydants, des solvants, des parfums, des charges, des filtres, des absorbeurs d'odeurs et des matières colorantes.

Les quantités des différents constituants des compositions décrites dans le présent texte sont celles classiquement utilisées dans les domaines considérés.

La quantité de composé chimique ou biologique ou de polypeptide, de séquence d'acides nucléiques ou d'agent modulateur contenue dans une composition du présent texte, encore dite « quantité efficace » est, bien entendu, fonction de la nature du composé et de l'effet recherché et peut donc varier dans une large mesure.

Pour donner un ordre de grandeur, une composition peut contenir un agent modulateur ou un polypeptide en une quantité représentant de 0,00001 % à 50 % du poids total de la composition, en particulier en une quantité représentant de 0,001 % à 10 % du poids total de la composition, et plus particulièrement en une quantité représentant de 0,1 % à 1 % du poids total de la composition.

Une composition décrite dans le présent texte peut être plus particulièrement dédiée à la réduction et/ou au traitement d'un défaut d'hydratation, et notamment une sécheresse, susceptible de détériorer l'état d'un épithélium, et notamment d'un épiderme.

Le présent texte décrit également l'utilisation d'au moins un polypeptide conforme à l'invention ou d'au moins une séquence d'acides nucléiques codant pour ledit polypeptide, à titre d'outil de caractérisation *in vitro* ou *ex vitro* d'un état de sécheresse d'un épithélium, et notamment d'un épiderme.

Ainsi, la présente invention concerne selon l'un de ses aspects des procédés non invasifs pour caractériser l'état de sécheresse de la surface d'un épiderme non pathologique ou encore l'efficacité d'un traitement cosmétique ou thérapeutique visant à caractériser qualitativement ou quantitativement l'expression de l'annexine II, de l'un de ses dérivés ou fragments.

Ces procédés sont particulièrement avantageux dans la mesure où leur mise en oeuvre ne nécessite pas de recourir obligatoirement à une technique opératoire pour procéder à une telle caractérisation. Un extrait de l'épiderme peut ainsi être obtenu par simple « stripping » et directement analysé par une technique d'analyse conventionnelle notamment telle que décrite précédemment.

Un procédé de caractérisation d'un état de sécheresse d'un épithélium, par exemple un épiderme, comprend au moins les étapes consistant à :
a) déterminer, dans un échantillon dudit épithélium, une teneur cfun polypeptide conforme à l'invention, ou d'une séquence d'acides nucléiques codant pour ledit polypeptide, et
b) comparer ladite teneur déterminée à l'étape a) à une valeur de référence.

Avantageusement, un procédé de l'invention est non invasif.

Un procédé de l'invention est avantageusement effectué sur un échantillon isolé.

Selon un mode de réalisation, un procédé selon l'invention peut être effectué sur un échantillon d'épithélium, et notamment d'épiderme, prélevé chez un individu.

Un procédé selon l'invention peut également être effectué sur un échantillon d'épithélium, et notamment d'épiderme, prélevé à partir d'un modèle cellulaire épithélial, et notamment épidermique, ou d'une peau isolée reconstruite afin d'en qualifier l'état.

Le prélèvement d'un échantillon d'épithélium peut se faire par toute méthode connue de l'homme de l'art.

Un procédé selon l'invention peut être effectué *in vivo, in vitro* ou *ex vivo.*

Une valeur de référence peut être, par exemple, une teneur en polypeptide ou en séquence d'acides nucléiques déterminée sur un échantillon d'épiderme prélevé sur un épithélium, et notamment une peau normale, c'est-à-dire satisfaisante sur le plan physiologique, à l'image par exemple d'une peau hydratée.

La mesure d'une valeur de référence peut être effectuée parallèlement ou séquentiellement à la détermination de ladite teneur d'un polypeptide ou d'une séquence d'acides nucléiques.

Une comparaison d'une teneur déterminée avec une valeur de référence peut permettre d'évaluer une déviation par rapport à cette valeur.

L'analyse de l'intensité et/ou de la nature de cette déviation (négative ou positive) peut être informative de l'état de l'épiderme.

La caractérisation d'un état de sécheresse d'un épiderme peut être indicative d'un éventuel trouble cutané qui peut être corrigé par l'utilisation de composés susceptibles de moduler l'expression d'un polypeptide de l'invention.

Selon un mode de réalisation, un procédé selon l'invention peut être mis en oeuvre dans un procédé de diagnostic *in vivo, in vitro* ou *ex vivo* de sécheresse d'un épithélium, et notamment de l'épiderme, chez un individu.

Un polypeptide convenant à la mise en oeuvre d'un procédé selon l'invention peut être avantageusement l'annexine II.

La détermination d'une teneur en polypeptide conforme à l'invention ou en acides nucléiques conformes à l'invention dans un échantillon d'épiderme peut être effectuée par tout protocole connu de l'homme de l'art.

L'expression d'une séquence d'acide nucléique peut être déterminée, par exemple, au moyen de sondes oligonucléotidiques, par tout protocole connu de l'homme de l'art.

A titre d'exemples de méthodes de détection de séquence d'acides nucléiques, il peut être fait mention de la réaction de polymérisation en chaîne, quantitative (Q-PCR) ou non (PCR), en présence (RT-PCR ou Q-RT-PCR) ou non de la transcriptase inverse, du Northern blot, de la méthode «ribonuclease protection assay », des méthodes avec des puces à ADN, des méthodes avec des puces transcriptomiques, des méthodes avec des puces à oligonucléotides, des méthodes d'hybridation *in situ.*

A titre d'exemple d'agents convenant à la détection d'une séquence d'acides nucléiques, et en particulier d'ARNm, il peut être fait mention de sondes d'acides nucléiques marquées pouvant s'hybrider à ladite séquence.

Une telle sonde d'acide nucléique peut être aisément obtenue par toute méthode connue de l'homme de l'art.

Ainsi, les séquences d'acides nucléiques conformes à l'invention peuvent être utilisées pour réaliser des amorces oligonucléotidiques sens et/ou anti-sens, qui s'hybrident dans des conditions de forte stringence à la séquence SEQ ID NO 1 ou un analogue de celle-ci.

L'expression d'une séquence d'acide nucléique conforme à l'invention peut être comparée à une valeur de référence obtenue, par exemple, en effectuant un procédé conforme à l'invention en l'absence de composé à tester.

L'expression d'une séquence d'acide nucléique peut également être déterminée, de manière indirecte, par la détermination de l'expression du polypeptide codé par ladite séquence, au moyen de toute technique connue dans le domaine, telle que le Western-Blot, l'ELISA, la méthode de Bradford ou de Lowry, ou comme indiqué ci-après.

Une séquence d'acide nucléique convenant à la mise en oeuvre d'un procédé selon l'invention peut être avantageusement une séquence d'acides nucléiques codant pour l'annexine II, par exemple de type ARNm.

La détermination d'une teneur d'un polypeptide conforme à l'invention peut être effectuée au moyen de toute méthode connue de l'homme de l'art.

A titre d'exemples de méthodes de détection d'un polypeptide, il peut être fait mention du Western-blot, du Slot-blot, du Dot-blot, des méthodes ELISA (Enzyme Linked Immuno-Sorbent Assay) de type singleplex ou multiplex, des méthodes de protéomique ou de glycomique, de coloration de polypeptides dans un gel de polyacrylamide par un colorant à base d'Argent, par le bleu de Coomassie ou par le SYPRO, de l'immunofluorescence, de l'absorption UV, de méthodes immunohistochimiques en microscopie classique, électronique ou confocale, de FRET (fluorescence résonance energy transfer/transfert d'énergie par résonance de fluorescence), des méthodes de TR-FRET (time resolved FRET/FRET en résolution de temps), des méthodes de FLIM (fluorescence lifetime imaging microscopy/imagerie par microscopie en temps de fluorescence), des méthodes de FSPIM (fluorescence spectral imaging microscopy/imagerie par microscopie en spectre de fluorescence), des méthodes de FRAP (fluorescence recovery after photobleaching/recouvrement de fluorescence après photoblanchiment), des méthodes par gène rapporteur, des méthodes d'AFM (atomic force microscopy/microscopie par force atomique), des méthodes de résonance plasmonique de surface, des méthodes de microcalorimétrie, des méthodes de cytométrie en flux, des méthodes de biosenseurs, des méthodes de radioimmuno-essais (RIA), des méthodes de focalisation isoélectrique, et des tests enzymatiques, des méthodes mettant en oeuvre des puces à peptides, des puces à sucre, des puces d'anticorps, des méthodes de spectrométrie de masse, des méthodes de spectrométrie de type SELDI-TOF (Ciphergen).

Les procédés conformes à l'invention peuvent être effectués sur un échantillon, par exemple isolé, d'épithélium, notamment d'épiderme, obtenu à partir d'une biopsie cutanée ou d'un modèle cellulaire épithélial, par exemple épidermique ou plus avantageusement d'un prélèvement de surface non invasif, notamment par adhésif (« tape stripping ») de *stratum corneum* ou par simple lavage.

Un prélèvement d'un échantillon d'épiderme peut se faire par toute méthode connue de l'homme de l'art.

Ces méthodes peuvent être effectuées par des techniques dites de « stripping ».

Ces strippings sont des surfaces collantes appliquées à la surface de l'épiderme comme le Blenderm^{®} de 3M, le D'squam (adhésif commercial de CuDERM), la colle cyanoacrylate ou la méthode du « stripping » vernis. Grâce à ces « strippings », les coméocytes adhérents et le contenu de leurs espaces intercellulaires peuvent être prélevés et soumis par la suite à une extraction permettant d'avoir accès au contenu protéique.

Le prélèvement d'un échantillon convenant au procédé peut aussi être effectué de façon plus dirècte par « lavages » de la surface cutanée, au moyen par exemple d'accessoires de type turbine à ailette, de type cellule à spirale (tel que décrit dans le brevet FR 2 667 778) associée à un circuit fluidique, ou simplement par ajout/prélèvement d'une goutte de tampon à la surface de la peau.

A titre indicatif, d'autres méthodes de prélèvement adaptées à la mise en oeuvre de l'invention peuvent être mentionnées, telles que des méthodes par grattage de la partie supérieure du *stratum corneum* au moyen d'un système bilames. Cette technique permet de recueillir des squames qui peuvent ensuite être directement analysées par différentes techniques pour déterminer les taux en minéraux, aminoacides ou lipides.

Egalement, il peut être envisagé de détecter la présence d'un polypeptide conforme à l'invention au moyen d'un anticorps, le cas échéant sous une forme marquée.

Un anticorps susceptible d'être utilisé à titre d'outil d'évaluation d'un état d'un épiderme peut être obtenu par tout procédé connu de l'homme de l'art, tel que décrit dans « Antibodies: A Laboratory Manual », Cold Spring Harbor Laboratory Press, Cold Spring Harbor, N.Y. (1990). De façon avantageuse, les anticorps utilisés peuvent être des anticorps recombinants, tels que ceux développés par la société Antibodies-by-design.

La présente invention concerne également un procédé, non thérapeutique, pour mettre en évidence un effet d'un traitement susceptible de faire régresser des signes de sécheresse d'un épithélium, notamment des sensations de démangeaisons et/ou de tiraillements, chez un individu, comprenant au moins les étapes consistant à :
a) effectuer, avant le traitement, au moins une première détermination, dans un premier échantillon d'un épithélium prélevé chez ledit individu, d'une activité biologique et/ou de l'expression d'un polypeptide conforme à l'invention, ou de l'expression d'une séquence d'acides nucléiques codant pour ledit polypeptide,
b) effectuer, après le traitement, au moins une seconde détermination, dans un second échantillon d'un épithélium prélevé chez ledit individu, de ladite activité biologique et/ou de ladite expression dudit polypeptide ou de ladite expression de ladite séquence d'acides nucléiques déterminées à l'étape a), et
c) comparer les première et deuxième déterminations, notamment afin d'en déduire une information relative à un effet au moins du traitement.

Un tel traitement peut en particulier être un traitement cosmétique.

En particulier, le traitement dont l'effet est à évaluer peut être un traitement destiné à soulager ou réduire les signes de sécheresse cutanée

L'activité biologique d'un polypeptide connue de l'homme de l'art, par exemple et de manière non limitative, on peut mentionner des méthodes de culture cellulaire suivie d'une caractérisation de marqueurs de différenciation, tels que par exemple la kératine 10, la filaggrine ou de marqueurs de prolifération tel que par exemple KI 67 et PCNA.

Avantageusement, un polypeptide mis en oeuvre dans un procédé selon la présente invention peut être l'annexine II.

L'expression d'un polypeptide peut être déterminée comme indiqué précédemment.

Le présent texte décrit un procédé de traitement cosmétique des signes de sécheresse cutanée comprenant au moins une étape consistant à appliquer sur une partie au moins de la peau, des muqueuses et/ou des fibres kératiniques, au moins une composition cosmétique décrite dans le présent texte.

Au sens de la présente invention, « un » doit se comprendre, sauf indication contraire, au sens de « au moins un ».

Les exemples figurant ci-après sont présentés à titre illustratif et non limitatif de l'invention.

### EXEMPLE I

### Analyse de l'expression de l'annexine II dans un stratum corneum sec vs. un stratum corneum normalement hydraté

Les analyses sont réalisées à partir d'échantillons prélevés par « strippings » vernis effectués sur les jambes de différents individus.

Les sujets se prêtant à l'étude sont regroupés en 4 groupes.

Le groupe AS correspond au groupe 1 : ménopausées sèches n=15.

Le groupe AN correspond au groupe 2 : ménopausées normales n=13.

Le groupe JS correspond au groupe 3 : jeunes sèches n=16.

Le groupe JN correspond au groupe 4 : jeunes normales n=14.

La sélection des sujets dans l'un ou l'autre groupe s'est faite sur la base de l'évaluation visuelle, par un expert, de l'étât de sécheresse cutanée de chaque individu au niveau des jambes.

La comparaison avec un atlas photographique a permis d'attribuer à chaque individu un score clinique de sècheresse cutanée selon le gradeur et l'échelle suivants :
- score 0 : peau normale ; la peau présente un relief cutané régulier et un aspect lisse,
- score 1 : peau déshydratée ; la peau présente un relief cutané strié et un aspect assez rêche,
- score 3 : peau très sèche ; la peau présente de nombreuses squames et quelques écailles, ainsi qu'un aspect rugueux, et
- score 4 : peau extrêmement sèche ; la peau présente de très nombreuses écailles et un aspect très rugueux.

### 1 : Préparation de poudres acétoniques

Deux « strippings » vernis (B. Mehul et al., J Biol Chem 2000, Apr 28; 275(17): 12841-7) de 10 cm² sont disposés dans 20 ml d'acétone. Les coméocytes se décollent. Le mélange est filtré sur membrane de nylon de 40 µm. Trois rinçages successifs sont effectués avec le même volume d'acétone. La suspension est finalement filtrée sur pompe à vide. On obtient des poudres acétoniques de *stratum corneum* sous forme sèche.

### 2 : Extraction des échantillons

On procède à une extraction en conditions dénaturantes. Pour ce faire, un prélavage est réalisé avec un volume (100 µl) de tampon PBS (Phosphate Buffer Saline) +0,1 % Triton X100 qui est ajouté par mg de poudres acétoniques. Le mélange est broyé au potter et centrifugé. Le culot de cornéocytes est recueilli. Il est extrait avec un même volume (100 µl/mg) de tampon Laemmli 0,0625 mM tris pH 6,8, 200 mM DIT, 2 % SDS, 10 % glycérol. Le mélange est porté à ébullition pendant 10 minutes, puis il est broyé et centrifugé 10 minutes à 10000 g. Le surnageant est recueilli. Un dosage de protéines est réalisé selon la technique de Bradford avec le réactif Bradford (Bio-Rad Protein assay) Les échantillons sont ajustés à 1 mg/ml.

### 3 : Analyses des protéines par Western-blot

Les protéines sont séparées par électrophorèse SDS-PAGE. Après transfert semi sec sur membrane de PVDF (Immobilon-P Milipore) selon un protocole standard, les protéines sont incubées avec l'anticorps primaire anti-annexine II (BD Biosciences, 610068) toute une nuit à 4 °C. Puis la seconde incubation a lieu avec l'anticorps secondaire (Goat anti mouse IgG-HRP conjugate ; Bio-Rad) dirigé contre l'anticorps primaire 1h30 à température ambiante. La présence de l'Annexine II sur la membrane est révélée par immunodétection à l'aide du kit ECL plus (Amersham). La membrane est ensuite colorée à l'Amidoblack pour détecter les protéines totales présentes sur la membrane. L'image est acquise au FluorSmax (Biorad) et les bandes quantifiées à l'aide du logiciel Quantity-one (Biorad).

### 4 : Résultats

Les résultats sont exprimés en delta cnt*mm² de la protéine d'intérêt/delta cnt*mm² des protéines totales.

### Méthodologie :

- analyse de variance à 2 facteurs Age et Type de peau prenant en compte l'interaction de ces deux facteurs + analyse de variance à 1 facteur (groupe) et test de comparaisons multiples de Tukey. Les conditions de normalité et d'homoscédasticité n'étant pas vérifiées, l'analyse a été réalisée après transformation logarithmique.

L'analyse statistique a été effectuée avec les logiciels SAS version 8.2 et SPSS version 12.

Tous les tests ont été réalisés au seuil bilatéral de 5 %.

Le tableau ci-après reprend les résultats moyennés ainsi que leurs écart-types à la moyenne (sem).

| groupe | Annexine II | sem Annexine II |
|---|---|---|
| **AS** | 441 | 165 |
| **AN** | 154 | 21 |
| **JS** | 221 | 55 |
| **JN** | 135 | 29 |

On note une variation significative de l'expression de l'annexine II selon la typologie de la peau : en effet, son expression est significativement accrue dans les groupes « peaux sèches » (JS et AS), comparativement aux groupes « peaux normales » (JN et AN) (p = 0,006).

### Liste de Séquences

**SEQ ID NO 1** 
1 gggacgctct cagctctcgg cgcacggccc aggttatctt gtagcatagc aacttcggat
61 ttcactctac ccggagagtt tcccgcttgg ttgaacacat tggcctcagg aagcttcctt
121 caaaatgtct actgttcacg aaatcctgtg caagctcagc ttggagggtg atcactctac
181 acccccaagt gcatatgggt ctgtcaaagc ctatactaac tttgatgctg agcgggatgc
241 tttgaacatt gaaacagcca tcaagaccaa aggtgtggat gaggtcacca ttgtcaacat
301 tttgaccaac cgcagcaatg cacagagaca ggatattgcc ttcgcctacc agagaaggac
361 caaaaaggaa cttgcatcag cactgaagtc agccttatct ggccacctgg agacggtgat
421 tttgggccta ttgaagacac ctgctcagta tgacgcttct gagctaaaag cttccatgaa
481 ggggctggga accgacgagg actctctcat tgagatcatc tgctccagaa ccaaccagga
541 gctgcaggaa attaacagag tctacaagga aatgtacaag actgatctgg agaaggacat
601 tatttcggac acatctggtg acttccgcaa gctgatggtt gccctggcaa agggtagaag
661 agcagaggat ggctctgtca ttgattatga actgattgac caagatgctc gggatctcta
721 tgacgctgga gtgaagagga aaggaactga tgttcccaag tggatcagca tcatgaccga
781 gcggagcgtg ccccacctcc agaaagtatt tgataggtac aagagttaca gcccttatga
841 catgttggaa agcatcagga aagaggttaa aggagacctg gaaaatgctt tcctgaacct
901 ggttcagtgc attcagaaca agcccctgta ttttgctgat cggctgtatg actccatgaa
961 gggcaagggg acgcgagata aggtcctgat cagaatcatg gtctcccgca gtgaagtgga
1021 catgttgaaa attaggtctg aattcaagag aaagtacggc aagtccctgt actattatat
1081 ccagcaagac actaagggcg actaccagaa agcgctgctg tacctgtgtg gtggagatga
1141 ctgaagcccg acacggcctg agcgtccaga aatggtgctc accatgcttc cagctaacag
1201 gtctagaaaa ccagcttgcg aataacagtc cccgtggcca tccctgtgag ggtgacgtta
1261 gcattacccc caacctcatt ttagttgcct aagcattgcc tggccttcct gtctagtctc
1321 tcctgtaagc caaagaaatg aacattccaa ggagttggaa gtgaagtcta tgatgtgaaa
1381 cactttgcct cctgtgtact gtgtcataaa cagatgaata aactgaattt gtactttaaa
1441 aaaaaaaaaa aaaaaa
**SEQ ID NO 2** 
1 MSTVHEILCK LSLEGDHSTP PSAYGSVKAY TNFDAERDAL NIETAIKTKG VDEVTIVNIL
61 TNRSNAQRQD IAFAYQRRTK KELASALKSA LSGHLETVIL GLLKTPAQYD ASELKASMKG
121 LGTDEDSLIE IICSRTNQEL QEINRVYKEM YKTDLEKDII SDTSGDFRKL MVALAKGRRA
181 EDGSVIDYEL IDQDARDLYD AGVKRKGTDV PKWISIMTER SVPHLQKVFD RYKSYSPYDM
241 LESIRKEVKG DLENAFLNLV QCIQNKPLYF ADRLYDSMKG KGTRDKVLIR IMVSRSEVDM
301 LKIRSEFKRK YGKSLYYYIQ QDTKGDYQKA LLYLCGGDD
**SEQ ID NO 3**
   AEDGSVIDYELIDQDAR
**SEQ ID NO 4**
   AYTNFDAER
**SEQ ID NO 5**
   AYTNFDAERDAL
**SEQ ID NO 6**
   DAERDALNIET
**SEQ ID NO 7**
   DALNIETAIK
**SEQ ID NO 8**
   DALNIETAIKTK
**SEQ ID NO 9**
   DIISDTSGDFR
**SEQ ID NO 10**
   DLYDAGVK
**SEQ ID NO 11**
   DLYDAGVKR
**SEQ ID NO 12**
   EGDHSTPPSAYGSVK
**SEQ ID NO 13**
   ELASALK
**SEQ ID NO 14**
   ELIDQDARDL
**SEQ ID NO 15**
   GDLENAFLNLVQCIQNKPLYFADR
**SEQ ID NO 16**
   GLGTDEDSLIEIICSR
**SEQ ID NO 17**
   GTDEDSLIEIICSR
**SEQ-ID NO 18**
   GVDEVTIVNI
**SEQ ID NO 19**
   GVDEVTIVNIL
**SEQ ID NO 20**
   GVDEVTIVNILTNR
**SEQ ID NO 21**
   HSTPPSAYGSVKA
**SEQ ID NO 22**
   HSTPPSAYGSVKAY
**SEQ ID NO 23**
   LMVALAK
**SEQ ID NO 24**
   LSLEGDHSTPPSAYGSVK
**SEQ ID NO 25**
   NKPLYFADR
**SEQ ID NO 26**
   QDIAFAYQR
**SEQ ID NO 27**
   RAEDGSVIDYELIDQDAR
**SEQ ID NO 28**
   SALSGHLETVIL
**SEQ ID NO 29**
   SALSGHLETVILGLLK
**SEQ ID NO 30**
   SEVDMLK
**SEQ ID NO 31**
   SLYYYIQQDTK
**SEQ ID NO 32**
   SLYYYIQQDTKGDYQK
**SEQ ID NO 33**
   STPPSAYGSVKAY
**SEQ ID NO 34**
   STPPSAYGSVKAYTNF
**SEQ ID NO 35**
   STVHEILCK
**SEQ ID NO 36**
   SYSPYDMLESIR
**SEQ ID NO 37**
   TDLEKDIISDTSGDFR
**SEQ ID NO 38**
   TNFDAERDALNIETAIKTK
**SEQ ID NO 39**
   TNQELQEINR
**SEQ ID NO 40**
   TPAQYDASELK
**SEQ ID NO 41**
   WISIMTER
**SEQ ID NO 42**
   YTNFDAER
**SEQ ID NO 43**
   YIQQDTKGDYQKAL

## Revendications

1. Procédé de caractérisation d'un état de sécheresse d'un épithélium comprenant au moins les étapes consistant à :
a) déterminer dans un échantillon dudit épithélium, une teneur d'un polypeptide de séquence d'acides aminés codée par une séquence d'acides nucléiques représentée par une séquence représentée par SEQ ID NO 1, un analogue de celle-ci ayant au moins 85 % d'homologie de séquence ainsi qu'une activité biologique de même nature, ou un fragment de celle-ci comprenant au moins 6 acides aminés consécutifs dudit polypeptide ainsi qu'une activité biologique de même nature ou d'une séquence d'acides nucléiques codant pour ledit polypeptide, et
b) comparer ladite teneur déterminée à l'étape a) à une valeur de référence.

2. Procédé selon la revendication précédente, **caractérisé en ce qu'**il est non invasif.

3. Procédé, non thérapeutique, pour mettre en évidence un effet d'un traitement susceptible de faire régresser des signes de sécheresse d'un épithélium, notamment des sensations de démangeaisons et/ou de tiraillements, chez un individu, comprenant au moins les étapes consistant à :
a) effectuer, avant le traitement, au moins une première détermination, dans un premier échantillon d'un épithélium prélevé chez ledit individu, d'une activité biologique et/ou de l'expression d'un polypeptide de séquence d'acides aminés codée par une séquence d'acides nucléiques représentée par une séquence représentée par SEQ ID NO 1, un analogue de celle-ci ayant au moins 85 % d'homologie de séquence ainsi qu'une activité biologique de même nature, ou un fragment de celle-ci comprenant au moins 6 acides aminés consécutifs dudit polypeptide ainsi qu'une activité biologique de même nature ou de l'expression d'une séquence d'acides nucléiques codant pour ledit polypeptide,
b) effectuer, après le traitement, au moins une seconde détermination, dans un second échantillon d'un épithélium prélevé chez ledit individu, de ladite activité biologique et/ou de ladite expression dudit polypeptide ou de ladite expression de ladite séquence d'acides nucléiques déterminées à l'étape a), et
c) comparer les première et deuxième déterminations, notamment afin d'en déduire une information relative à un effet au moins du traitement.

4. Procédé selon l'une quelconque des revendications 1 à 3, dans laquelle ledit polypeptide a une séquence d'acides aminés représentée par une séquence représentée par SEQ ID NO 2, un analogue de celle-ci ayant au moins 85 % d'homologie de séquence ainsi qu'une activité biologique de même nature, ou un fragment de celle-ci comprenant au moins 6 acides aminés consécutifs dudit polypeptide ainsi qu'une activité biologique de même nature.

5. Procédé selon l'une quelconque des revendications 1 à 4, dans laquelle ledit polypeptide a une séquence d'acides aminés choisie parmi SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24, SEQ ID NO 25, SEQ ID NO 26, SEQ ID NO 27, SEQ ID NO 28, SEQ ID NO 29, SEQ ID NO 30, SEQ ID NO 31, SEQ ID NO 32, SEQ ID NO 33, SEQ ID NO 34, SEQ ID NO 35, SEQ ID NO 36, SEQ ID NO 37, SEQ ID NO 38, SEQ ID NO 39, SEQ ID NO 40, SEQ ID NO 41, SEQ ID NO 42 et SEQ ID NO 43.

## Patentansprüche

1. Verfahren zur Charakterisierung eines Trockenheitszustandes eines Epithels mindestens umfassend die folgenden Schritte:
a) Bestimmen in einer Probe des Epithels eines Gehalts eines Polypeptids der Aminosäuresequenz, die von einer Nukleinsäuresequenz kodiert wird, die durch eine durch SEQ ID NO: 1 dargestellte Sequenz dargestellt wird, eines Analogons davon mit mindestens 85 % Sequenzhomologie sowie einer biologischen Aktivität der gleichen Art, oder eines Fragments davon, das mindestens 6 aufeinanderfolgende Aminosäuren des Polypeptids sowie eine biologische Aktivität der gleichen Art umfasst, oder einer Nukleinsäuresequenz, die das Polypeptid kodiert, und
b) Vergleichen des in Schritt a) bestimmten Gehalts mit einem Referenzwert.

2. Verfahren nach dem vorhergehenden Anspruch, **dadurch gekennzeichnet, dass** es nicht invasiv ist.

3. Nichttherapeutisches Verfahren zum Nachweisen einer Wirkung einer Behandlung, die dazu geeignet ist, Anzeichen von Trockenheit eines Epithels, insbesondere Gefühle von Juckreiz und/oder Spannungen bei einem Individuum zurückzudrängen, mindestens umfassend die folgenden Schritte:
a) Durchführen, vor der Behandlung, von mindestens einer ersten Bestimmung in einer ersten Probe von einem dem Individuum entnommenen Epithel einer biologischen Aktivität und/oder der Expression eines Polypeptids der Aminosäuresequenz, die von einer Nukleinsäuresequenz kodiert wird, die durch eine durch SEQ ID NO: 1 dargestellte Sequenz dargestellt wird, eines Analogons davon mit mindestens 85 % Sequenzhomologie sowie einer biologischen Aktivität der gleichen Art, oder eines Fragments davon, das mindestens 6 aufeinanderfolgende Aminosäuren des Polypeptids sowie eine biologische Aktivität der gleichen Art umfasst, oder der Expression einer Nukleinsäuresequenz, die das Polypeptid kodiert,
b) Durchführen, nach der Behandlung, von mindestens einer zweiten Bestimmung in einer zweiten Probe von einem dem Individuum entnommenen Epithel der biologischen Aktivität und/oder der Expression des Polypeptids oder der Expression der Sequenz von in Schritt a) bestimmten Nukleinsäuren, und
c) Vergleichen der ersten und zweiten Bestimmung, insbesondere um daraus eine Information bezüglich einer Wirkung von mindestens der Behandlung abzuleiten.

4. Verfahren nach einem der Ansprüche 1 bis 3, wobei das Polypeptid eine Aminosäurequenz aufweist, die durch eine durch SEQ ID NO 2 dargestellte Sequenz, ein Analogon davon mit mindestens 85 % Sequenzhomologie sowie einer biologischen Aktivität der gleichen Art, oder ein Fragment davon, das mindestens 6 aufeinanderfolgende Aminosäuren des Polypeptids sowie eine biologische Aktivität der gleichen Art umfasst, dargestellt wird.

5. Verfahren nach einem der Ansprüche 1 bis 4, wobei das Polypeptid eine Aminosäurequenz aufweist, die ausgewählt ist aus SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24, SEQ ID NO 25, SEQ ID NO 26, SEQ ID NO 27, SEQ ID NO 28, SEQ ID NO 29, SEQ ID NO 30, SEQ ID NO 31, SEQ ID NO 32, SEQ ID NO 33, SEQ ID NO 34, SEQ ID NO 35, SEQ ID NO 36, SEQ ID NO 37, SEQ ID NO 38, SEQ ID NO 39, SEQ ID NO 40, SEQ ID NO 41, SEQ ID NO 42 und SEQ ID NO 43.

## Claims

1. A method for characterizing a state of dryness of an epithelium comprising at least the steps of:
a) determining in a sample of said epithelium, a content of a polypeptide with a sequence of amino acids coded by a sequence of nucleic acids represented by a sequence represented by SEQ ID No. 1, an analog of the latter having at least 85% sequence homology as well as a biological activity of the same nature, or a fragment of the latter comprising at least 6 consecutive amino acids of said polypeptide as well as a biological activity of the same nature, or of a sequence of nucleic acids coding for said polypeptide, and
b) comparing said content determined in step a) with a reference value.

2. The method according to the preceding claim, **characterized in that** it is non-invasive.

3. A non-therapeutic method for showing an effect of a treatment which may cause a regression of signs of dryness of an epithelium, notably sensations of itching and/or pulling, in an individual, comprising at least the steps of:
a) carrying out, before treatment, at least one first determination, in a first sample of an epithelium taken from said individual, of a biological activity and/or of the expression of a polypeptide with a sequence of amino acids coded by a sequence of nucleic acids represented by a sequence represented by SEQ ID No. 1, an analog of the latter having at least 85% sequence homology as well as a biological activity of the same nature, or a fragment of the latter comprising at least 6 consecutive amino acids of said polypeptide as well as a biological activity of the same nature, or of the expression of a sequence of nucleic acids coding for said polypeptide,
b) carrying out, after the treatment, at least a second determination, in a second sample of an epithelium taken from said individual, of said biological activity and/or of said expression of said polypeptide or of said expression of said sequence of nucleic acids determined in step a), and
c) comparing the first and second determinations, notably in order to infer therefrom an information relating to at least one effect of the treatment.

4. The method according to any of claims 1 to 3, wherein said polypeptide has a sequence of amino acids represented by a sequence represented by SEQ ID No. 2, an analog of the latter having at least 85% sequence homology as well as a biological activity of the same nature, or a fragment of the latter comprising at least 6 consecutive amino acids of said polypeptide as well as a biological activity of the same nature.

5. The method according to any of claims 1 to 4, wherein said polypeptide has a sequence of amino acids selected from SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27, SEQ ID No. 28, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39, SEQ ID No. 40, SEQ ID No. 41, SEQ ID No. 42 and SEQ ID No. 43.
